(19) **Europäisches Patentamt**
**European Patent Office**
**Office européen des brevets**

(11) Veröffentlichungsnummer : **0 439 030 B1**

(12) # EUROPÄISCHE PATENTSCHRIFT

(45) Veröffentlichungstag der Patentschrift :
**31.03.93 Patentblatt 93/13**

(51) Int. Cl.$^5$ : **A61K 31/50,** A61K 9/20,
A61K 47/12

(21) Anmeldenummer : **91100309.3**

(22) Anmeldetag : **11.01.91**

(54) **Orale Arzneimittelformen von Pimobendan.**

(30) Priorität : **20.01.90 DE 4001622**

(43) Veröffentlichungstag der Anmeldung :
**31.07.91 Patentblatt 91/31**

(45) Bekanntmachung des Hinweises auf die
Patenterteilung :
**31.03.93 Patentblatt 93/13**

(84) Benannte Vertragsstaaten :
**AT BE CH DE DK ES FR GB GR IT LI LU NL SE**

(56) Entgegenhaltungen :
**EP-A- 0 008 391**
**EP-A- 0 306 846**
**EP-A- 0 330 052**
**DE-A- 3 728 244**

(73) Patentinhaber : **Dr. Karl Thomae GmbH**
**Postfach 1755**
**W-7950 Biberach 1 (DE)**

(72) Erfinder : **Gruber, Peter, Dr. Dipl.-Chem.**
**Stallenrain 14**
**CH-4103 Bottmingen (CH)**
Erfinder : **Roth, Willy, Dr. Dipl.-Chem.**
**Matthias-Erzberger-Strasse 35**
**W-7950 Biberach 1 (DE)**
Erfinder : **Schepky, Gottfried, Dr.**
**Ulrich-von-Hutten-Weg 2**
**W-7950 Biberach 1 (DE)**

EP 0 439 030 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

EP 0 439 030 B1

## Beschreibung

Die Erfindung betrifft oral zu applizierende Arzneimittelformen des Pimobendans. Pimobendan ist das 4,5-Dihydro-6-[2-(4-methoxyphenyl)-1H-benzimidazol-5-yl]-5-methyl-3(2H)-pyridazinon, welches in der Europäischen Patentschrift Nr. 0 008 391 beschrieben wurde; das Pimobendan ist eine kardiotonisch, blutdrucksenkend und antithrombotisch wirkende Substanz.

Im Gegensatz zu anderen in dieser Patentschrift genannten Substanzen mit abweichenden Strukturen unterliegt die Resorption von Pimobendan bei oraler Verabreichung starken inter- und intraindividuellen Schwankungen, sofern die Wirksubstanz in an sich bekannte bzw. übliche, oral zu verabreichende Arzneimittelformen eingearbeitet ist. Die Ursache hierfür ist, daß Pimobendan durch eine geringe Löslichkeit in wäßrigem Milieu und durch eine sehr hohe pH-Abhängigkeit der Löslichkeit charakterisiert ist.

Je nach dem verwendeten Puffersystem lösen sich im pH-Bereich zwischen 1 und 3 ca. 100 bis 300 mg/l (entspricht einer 0,01 bis 0,03%-igen Lösung), bei pH 5 jedoch nur noch ca. 1 mg/l (entspricht einer 0,0001%-igen Lösung bzw. 1 ppm) in Wasser auf.

Bei in-vivo-Versuchen am Menschen mit in Hartgelatine-Kapseln abgefülltem Pimobendan zeigte ein Proband keinen, ein zweiter Proband sehr niedrige, ein dritter höhere, insgesamt aber interindividuell sehr stark schwankende und zu niedrige Blutspiegel an Pimobendan. Dieses unbefriedigende Resorptionsverhalten kann im wesentlichen durch die hohe pH-Abhängigkeit der Löslichkeit des Pimobendans in wäßrigem Milieu und durch schwankende pH-Verhältnisse im Magen-Darm-Trakt der Probanden erklärt werden. Es ist bekannt, daß der pH-Wert des Magensaftes, insbesondere bei nüchternen Patienten, zwischen 1 und 6 schwanken kann, bei nicht nüchternen Patienten liegt er häufiger zwischen 3 und 5 als bei 1 bis 2.

Es lag nun nahe, die Löslichkeit von Pimobendan durch gleichzeitige Zugabe einer Säure zu erhöhen. In-vitro-Versuche zeigten aber, daß Pimobendan in einer 0,1 N Salzsäure (pH-Wert 1,1) sich nur mit 100 mg/l (entspricht einer 0,01%-igen Lösung) löst. In einer Fumarsäurelösung vom pH-Wert 2,27 lösen sich nur 50 mg/l (entspricht einer 0,005%-igen Lösung), in einer 20%-igen (Gew.-%) Weinsäurelösung vom pH-Wert 1,2 lösen sich 960 mg/l (entspricht einer 0,096%-igen Lösung), in einer 40%-igen Weinsäurelösung vom pH-Wert 0,7 nur 3,9 g/l (entspricht einer 0,39%-igen Lösung). Alle diese Werte reichen nicht aus bzw. der notwendige Säurezusatz ist nicht mehr praktikabel, um eine genügende Menge des Wirkstoffes in Lösung zu bringen und damit eine gesicherte Resorption zu gewährleisten, auch wenn entsprechende Mengen dieser Säuren zusammen mit dem Wirkstoff gleichzeitig oral verabreicht werden.

Überraschenderweise ist es jetzt gelungen, die geringe Löslichkeit und die hohe pH-Abhängigkeit der Löslichkeit des Pimobendans dadurch zu überwinden und eine sehr gute, gleichbleibendere Resorption auch bei Vorliegen starker pH-Schwankungen im Magen-Darm-Trakt zu gewährleisten, daß das Pimobendan mit Zitronensäure in einem Gewichtsverhältnis von höchstens oder kleiner als 1:5 innig vermischt und anschließend mit üblichen Hilfsstoffen zu einem oral zu verabreichenden Pulvern, Pellets oder Granulate verarbeitet wird. Die Granulate, Pulver oder Pellets können auch mit entsprechenden Hilfsstoffen zu Tabletten verpreßt werden, die man gewünschtenfalls noch mit einem geschmacksabdeckenden Überzug versieht.

Zitronensäure ist ein sicherer und gut verträglicher Hilfsstoff, der die Löslichkeit des Pimobendans um den Faktor 100 im Vergleich zu künstlichem Magensaft (pH 1,2) erhöht. So lösen sich 7,6 g/l in einer 20 Gew.-% Zitronensäure enthaltenden wäßrigen Lösung von pH 1,4, in einer 40 Gew.-% Zitronensäure enthaltenden wäßrigen Lösung von pH 1,0 sogar 12,1 g Pimobendan pro Liter auf. Diese Mengen an gelöstem Pimobendan reichen aber aus, um eine ausreichende Resorption des Wirkstoffes auch bei Patienten sicherzustellen, die nach oraler Verabreichung von herkömmlichen, Pimobendan enthaltenden Zubereitungsformen keine oder nur sehr niedrige und auch stark schwankende Blutspiegelwerte aufweisen.

Zitronensäure läßt sich schlecht zu festen Darreichungsformen verarbeiten. Um eine Salzbildung von Pimobendan mit Zitronensäure, und damit eine Steigerung der Hygroskopizität der Formulierung zu vermeiden, liegt es zunächst nahe, Wirkstoff und Säure in zwei getrennten Granulaten zu verarbeiten. Dabei hat es sich aber gezeigt (es sei hier auf die Beispiele 1b bis 1d und 2a verwiesen), daß bei dieser Trennung die Zitronensäure ihre lösungsvermittelnde Wirkung nur unvollständig entfalten kann. Es wurde aber gefunden, daß durch innige Vermischung von Pimobendan mit Zitronensäure unter Bildung eines Pulvergemisches, das anschließend zu einem Granulat, einem Pellet oder zu Tabletten verarbeitet wird, Darreichungsformen mit geringen Zitronensäuremengen möglich sind, die ausreichendes Auflösungsverhalten und ausreichende Blutspiegel gewährleisten. Technisch kann das z. B. durch eine nicht-wäßrige Granulation erreicht werden, z. B. durch eine Granulation mit Alkohol oder auch durch Anwendung entsprechender Granulierverfahren, die eine genau dosierte Granulierflüssigkeitszugabe unter gleichzeitiger Trocknung ermöglichen. Eine weitere Möglichkeit bietet die Herstellung eines Granulates durch Trockengranulation, welches Wirkstoff und Zitronensäure innig vermischt enthält. Wegen der hygroskopischen Eigenschaften der Zitronensäure ist darauf zu achten, daß die Zubereitungsformen im Freigabemedium rasch zerfallen; dies wird im Falle von Tabletten durch Zusatz

2

von Sprengmitteln bewirkt, z. B. von Amberlite® IRP 88 (Methacrylharz mit austauschbarem Proton), Crospovidone® (quervernetztes Polyvinylpyrrolidon) und mikrokristalline Cellulose, die gleichzeitig die schlechten Preßeigenschaften der Zitronensäure verbessert.

Bevorzugt wird ein Gewichtsverhältnis von Pimobendan zu Zitronensäure zwischen 1:10 und 1:20. Die obere Grenze wird durch die Schluckbarkeit der hergestellten Dareichungsformen limitiert.

Die Verhinderung von stark schwankenden Blutspiegelwerten (inter- und intraindividuell) durch Zugabe von Zitronensäure läßt sich wie folgt erklären: wenn das innige Gemisch aus Wirkstoff und Zitronensäure mit Magensaft in Berührung kommt, entsteht um die Partikel aufgrund der hohen Lösungsgeschwindigkeit der Zitronensäure eine saure Mikrosphäre. Diese Mikrosphäre ist stets sauer, unabhängig vom pH-Wert des Magen-Darm-Saftes und stellt sicher, daß der feinverteilte Wirkstoff sich zuverlässig auflöst und damit ungehindert zur Resorption zur Verfügung steht.

Durch Löslichkeitstests läßt sich für pH-Werte zwischen 1 und 6 nachweisen, daß sich der Wirkstoff aus diesem innigen Gemisch praktisch pH-unabhängig auflöst. Hinzu kommt, daß der Wirkstoff zusätzlich mit der Zitronensäure über Stunden stabile, übersättigte Lösungen bildet. Dadurch wird für jeden Fall ein hoher Grad an Resorption garantiert, auch bei Patienten mit abnormal hohen pH-Werten ihres Magen-Darm-Saftes. Unter den vielen für diese Zwecke geprüften Säuren nimmt die Zitronensäure eine unerwartete Sonderstellung ein; neben ihrer Funktion als Säure wirkt sie als Lösungsvermittler und gleichzeitig als ein Stabilisator der vermittelten Wirkstofflösung. Eine wichtige Voraussetzung für das Inlösungbringen des Wirkstoffes unabhängig vom örtlichen physiologischen pH-Wert ist die innige Vermischung des Pimobendans mit der Zitronensäure. Hierzu ist es erforderlich, daß beide Stoffe pulverförmig bzw. als sehr kleine Kristalle vorliegen, so daß sie sich mit einer großen Oberfläche gegenseitig berühren.

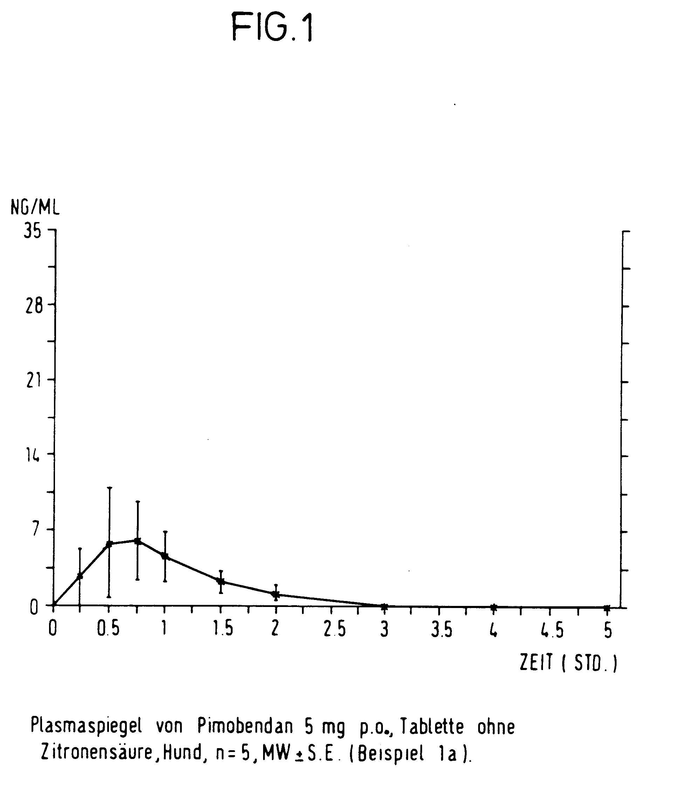

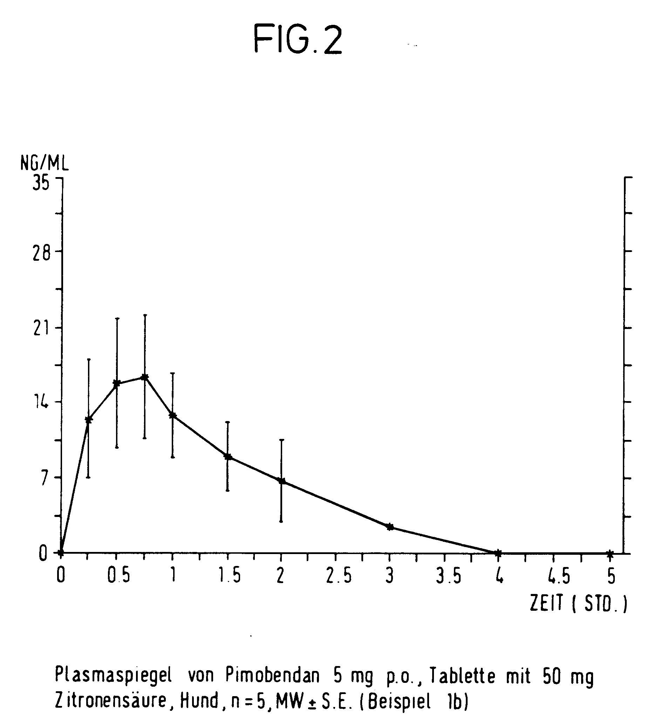

Versuche am Hund zeigten nach oraler Verabreichung einer Form gemäß Beispiel 1a mit 5 mg Pimobendan im Vergleich zu einer Form gemäß Beispiel 1b mit 5 mg Pimobendan und 50 mg Zitronensäure, daß sich der Pimobendan-Plasmaspiegel bei der die Zitronensäure enthaltenden Form gegenüber der Form ohne Zitronensäure annähernd verdreifacht hatte. Die Versuche wurden jeweils an 5 Versuchstieren durchgeführt. Die dabei gefundenen, gemittelten Kurvenwerte sind aus den Figuren 1 und 2 ersichtlich. Hierbei sind die Plasmaspiegelwerte in Nanogramm pro Milliliter gegenüber der Zeit aufgetragen.

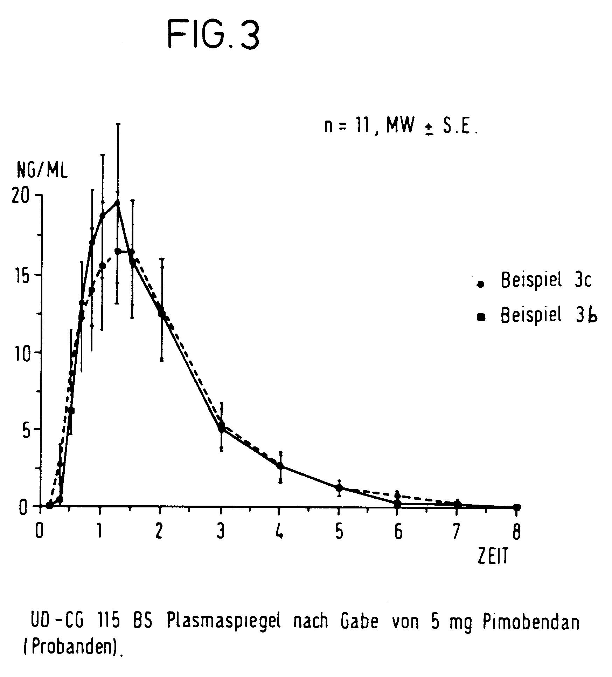

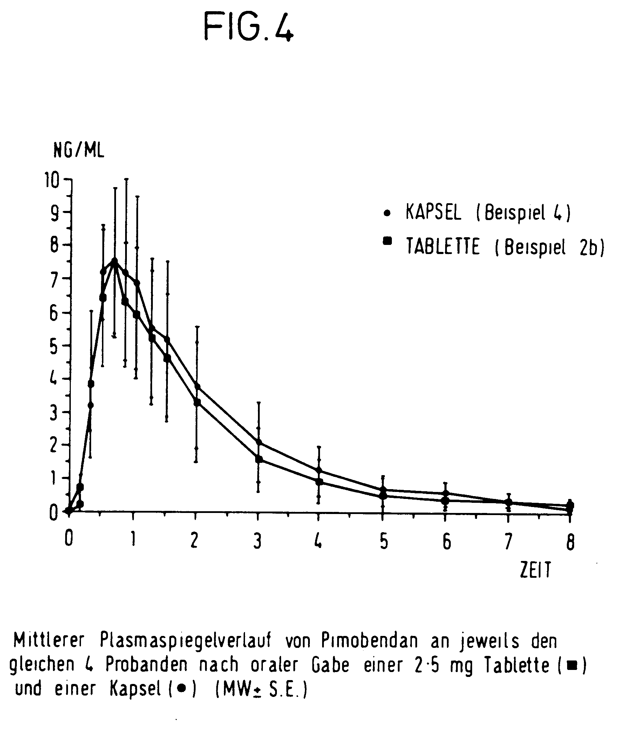

Humanuntersuchungen mit oralen Pimobendanformen gemäß den Beispielen 3b und 3c (Kapseln) an 11 Probanden ergaben die in der Figur 3 gezeigten mittleren Kurvenverläufe der Plasmaspiegel. Die Maxima liegen bei 1 bis 1,5 Stunden nach der Applikation. Zusätzlich zu den Kapselformulierungen gemäß den Beispielen 3b und 3c wurden noch die Plasmaspiegelverläufe der Tablettenform gemäß dem Beispiel 2b und der Kapselform gemäß dem Beispiel 4 an jeweils 11 Probanden untersucht. Hierbei stellte sich heraus, daß die Tablette mit nur 50 mg Zitronensäure gemäß Beispiel 2b mit der Kapselformulierung mit 209 mg Zitronensäure gemäß Beispiel 4 bioäquivalent ist. Die Plasmaspiegelwerte wurden mittels hochdruckflüssigkeitschromatographischen Methoden ermittelt, wobei sich die in Figur 4 gezeigten mittleren Kurvenverläufe der Plasmaspiegel ergaben (Mittelwerte ± Standard Deviations).

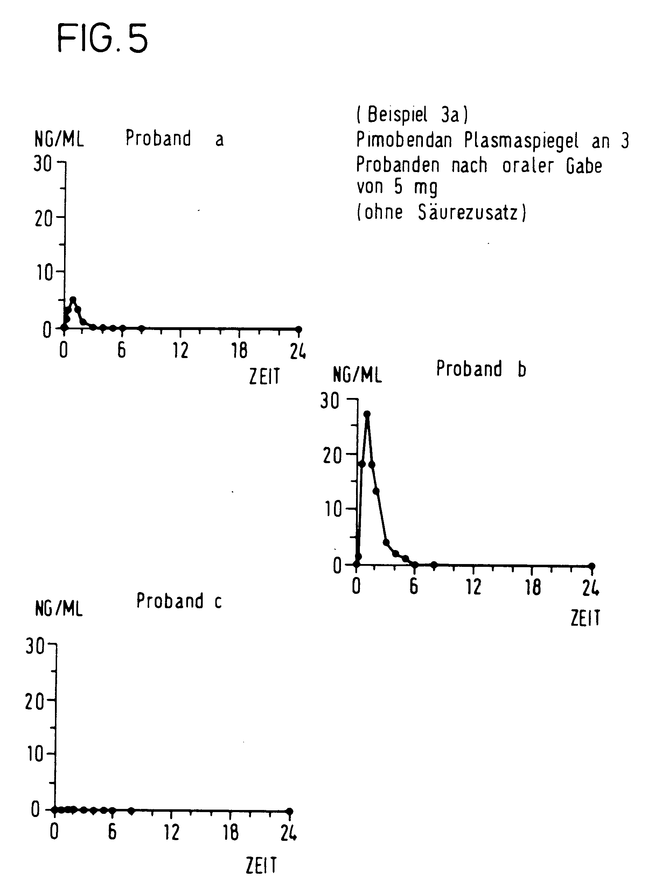

Zum Vergleich wurde eine Tabletten-Formulierung gemäß Beispiel 3a oral verabreicht, also eine Formulierung ohne Zitronensäure. Hierbei ergaben sich die wie in Figur 5a bis c aufgezeigten Kurvenverläufe der Plasmaspiegelwerte an drei Probanden. Vergleicht man die Figur 5a bis c mit der Figur 4 so wird die Überlegenheit der Zitronensäureform, die sich in geringeren Schwankungen der Plasmaspiegel bemerkbar macht, gegenüber einer Form ohne Zitronensäure sehr deutlich.

Es versteht sich von selbst, daß anstelle von Pimobendan auch eines seiner möglichen Enantiomeren mit dem gleichen Erfolg verwendet werden kann.

Zur näheren beispielhaften Erläuterung des Gegenstands der vorliegenden Erfindung sei auf die nachfolgenden Beispiele für orale Zubereitungsformen hingewiesen, hierbei bedeuten

Amberlite® IRP 88 = Methacrylharz mit austauschbarem $H^+$
Kollidon® 25 = Polyvinylpyrrolidon, mittleres Molekulargewicht 25 000
Avicel® = mikrokristalline Cellulose
Polyplasdone® XL = kreuzvernetztes Polyvinylpyrrolidon = Polyvinylpolypyrrolidon
Compritol® 888 = Glycerinmonobehenat
Tween® 80 = Polyoxyethylen-(20)-sorbitanmonooleat
Explotab® = Natriumcarboxymethylstärke
Aerosil® 130 V = hochdisperses, röntgenamorphes Siliciumdioxid.

Beispiel 1

Tabletten zu 5 mg Pimobendan

a.) Tabletten ohne Zitronensäure

Zusammensetzung:

```
      1 Tablette enthält (mg)

      (1)  Pimobendan                        5,0
      (2)  mikrokristalline Cellulose       58,0
      (3)  Calciumphosphat, sek.            72,0
      (4)  Maisstärke                       54,0
      (5)  Amberlite® IRP 88                10,0
      (6)  Magnesiumstearat         .        1,0
                                           200,0
```

Herstellung:

Ein Teil der Maisstärke wird in Wasser unter Erwärmen gelöst und damit die Mischung aus (1) bis (4) granuliert. Dem getrockneten Granulat werden (5) und (6) zugemischt. Aus der fertigen Mischung werden Tabletten von 8 mm Durchmesser und 200 mg Gewicht gepreßt.

Bestimmung der Auflösegeschwindigkeit:
Nach USP XXII, Paddle-Methode, 150 U/min, in McIlvaine-Puffer von pH 5,5.
$\bar{x}$ aus jeweils 3 Einzelbestimmungen.

Ergebnis:

```
      Nach  5 min:  8,5 %
           10 min: 10,2 %
           15 min: 10,7 %
           20 min: 10,8 %
           30 min: 10,8 % Pimobendan gelöst.
```

b.) Tabletten mit 50 mg Zitronensäure

Zusammensetzung:

```
         1 Tablette enthält (mg)


   (1)   Pimobendan                      5,0
   (2)   Zitronensäure                  50,0
   (3)   mikrokristalline Cellulose    42,0
   (4)   Kollidon® 25                    0,5
   (5)   Calciumphosphat, sek.         52,0
   (6)   Maisstärke                    39,5
   (7)   Amberlite® IRP 88             10,0
   (8)   Magnesiumstearat             __1,0
                                      200,0
```

Herstellung:

Ein Teil der Maisstärke wird in Wasser unter Erwärmen gelöst und damit (1), ein Teil von (3), (5) und ein Teil von (6) granuliert. (2) und die Reste von (3) und (6) werden mit der wäßrigen Lösung von (4) granuliert. Die Granulate werden getrocknet und miteinander vermischt. Der Mischung der getrockneten Granulate wird (7) und (8) zugemischt = Endmischung. Letztere wird zu Tabletten von 8 mm Durchmesser und 200 mg Gewicht verpreßt.

Bemerkung:

Wirkstoff und Säure liegen zur leichteren Herstellbarkeit in getrennten Granulaten vor, die aber miteinander vermischt werden.
Bestimmung der Auflösegeschwindigkeit: analog Beispiel 1a.

Ergebnis:

```
   Nach  5 min:  7,7 %
        10 min: 19,2 %
        15 min: 34   %


        20 min: 40,6 %
        30 min: 43   %   Pimobendan gelöst.
```

c.) Tabletten mit 103 mg Zitronensäure

Zusammensetzung:

1 Tablette enthält (mg)

| | | | |
|---|---|---|---|
| (1) | Pimobendan | | 5,0 |
| (2) | Zitronensäure | | 103,0 |
| (3) | mikrokristalline Cellulose | | 35,0 |
| (4) | Kollidon® 25 | | 1,0 |
| (5) | Calciumphosphat, sek. | | 31,5 |
| (6) | Maisstärke | | 81,5 |
| (7) | Amberlite® IRP 88 | | 10,0 |
| (8) | Magnesiumstearat | | 3,0 |
| | | | 270,0 |

Herstellung:

Analog zu Beispiel 1b.
Tabletten: 9 mm Durchmesser bei 270 mg Gewicht.

Bemerkung:

Wirkstoff und Säure liegen zur leichteren Herstellbarkeit in getrennten Granulaten vor, die aber miteinander vermischt werden.

d.) Tabletten mit 206 mg Zitronensäure

Zusammensetzung:

1 Tablette enthält (mg)

| | | | |
|---|---|---|---|
| (1) | Pimobendan | | 5,0 |
| (2) | Zitronensäure | | 206,0 |
| (3) | Avicel® | | 50,0 |
| (4) | Kollidon® 25 | | 2,0 |
| (5) | Calciumphosphat, sek. | | 63,0 |
| (6) | Maisstärke | | 46,0 |
| (7) | Amberlite® IRP 88 | | 20,0 |
| (8) | Magnesiumstearat | | 3,0 |
| | | | 395,0 |

Herstellung:

Analog zu Beispiel 1b.

Tabletten: 11 mm Durchmesser bei 395 mg Gewicht.

Bemerkung:

Wirkstoff und Säure liegen zur leichteren Herstellbarkeit in getrennten Granulaten vor, die aber miteinander vermischt werden.

Bestimmung der Auflösegeschwindigkeit: analog zu Beispiel 1a.

Ergebnis:

```
Nach  5 min: 23,8 %
     10 min: 59 %
     15 min: 67 %
     30 min: 69 %   Pimobendan gelöst.
```

Beispiel 2

Tabletten zu 2,5 mg Pimobendan

a.) Tabletten mit 103 mg Zitronensäure

Zusammensetzung:

```
1 Tablette enthält (mg)
```

| | | |
|---|---|---|
| (1) | Pimobendan | 2,5 |
| (2) | Maisstärke | 23,0 |
| (3) | mikrokrist. Cellulose | 26,0 |
| (4) | Calciumphosphat, wasserfr. | 31,5 |
| (5) | Polyplasdone® XL | 59,0 |
| (6) | Zitronensäure, Feingrieß (anhydro) | 103,0 |
| (7) | Compritol® 888 | 5,0 |
| | | 250,0 |

Herstellung:

(1) bis (4) werden mit wäßriger Stärkelösung granuliert. Dem getrockneten Granulat werden die übrigen Tablettenbestandteile zugemischt = Endmischung. Daraus werden Tabletten von 9 mm Durchmesser und 250 mg Gewicht gepreßt.

Bemerkung:

Wirkstoff und Säure liegen zur leichteren Herstellbarkeit getrennt voneinander vor, werden aber anschließend miteinander vermischt.

Bestimmung der Auflösegeschwindigkeit: analog Beispiel 1a.

Ergebnis:

```
Nach  5 min: 18,7 %
     10 min: 20,5 %
     20 min: 21,8 %
     30 min: 22,2 %
     60 min: 22,7 %    Pimobendan gelöst.
```

b. Tabletten mit 50 mg Zitronensäure

Zusammensetzung:

```
1 Tablette enthält (mg)

(1)  Pimobendan                              2,5
(2)  Zitronensäure pulv., wasserfrei        50,0
(3)  Avicel® PH 101                         13,0
(4)  Calciumhydrogenphosphat, wasserfrei    15,0
(5)  Maisstärke, ungetr.                     6,0
(6)  Kollidon® 25                            0,5
(7)  Polyvinylpyrrolidon, unlöslich         59,0
(8)  Compritol® 888                          3,0
(9)  Magnesiumstearat                        1,0
                                           150,0
```

Herstellung:

(6) wird in Ethanol gelöst und damit die Mischung aus (1) bis (5) granuliert. Dem getrockneten Granulat werden (7) bis (9) zugemischt = preßfertige Mischung. Letztere wird zu Tabletten von 8 mm Durchmesser verpreßt.

Bemerkung:

Wirkstoff und Säure liegen gemeinsam im selben Granulat vor.
Bestimmung der Auflösegeschwindigkeit: analog zu Beispiel 1a.

Ergebnis:

```
Nach 15 min: 71,1 %
     30 min: 85 %    Pimobendan gelöst.
```

Beispiel 3

Kapseln zu 5 mg Pimobendan

a.) Kapseln ohne Zitronensäure

Zusammensetzung:

```
          1 Kapsel enthält (mg)


    Pimobendan              5,0
    Milchzucker            90,25
    Maisstärke             36,0
    Tween®80                0,5
    Explotab®               8,0
    Magnesiumstearat        0,25
                          140,0
```

Herstellung:

Die einzelnen Pulver werden miteinander intensiv gemischt und in Hartgelatinekapseln der Größe 4 (140 mg/Kapsel) abgefüllt.

b.) Kapseln mit 230 mg Zitronensäure

Zusammensetzung:

```
          1 Kapsel enthält (mg)


    (1)  Pimobendan            5,0
    (2)  Zitronensäure       230,45
    (3)  Kollidon®25           3,78
    (4)  Magnesiumstearat      0,77
                             240,00 mg
```

Herstellung:

(1) und (2) werden intensiv miteinander gemischt und mit einer alkoholischen Lösung von (3) granuliert. Dem getrockneten Granulat wird (4) zugemischt. Die so erhaltene Endmischung wird in Hartgelatinekapseln der Größe 1 (240 mg/Kapsel) gefüllt.

Bemerkung:

Wirkstoff und Säure liegen in ein und demselben Granulat zusammen vor.
Bestimmung der Auflösegeschwindigkeit: analog zu Beispiel 1a.

Ergebnis:

Nach 5 min: 100 % Pimobendan gelöst.

c.) Kapseln mit 207 mg Zitronensäure

Zusammensetzung:

```
           1 Kapsel enthält (mg)


    (1)    Pimobendan, fein gemahlen          5,0
    (2)    Zitronensäure                    206,5
    (3)    mikrokrist. Cellulose             40,0
    (4)    Aerosil® 130 V                    11,0
    (5)    Kollidon® 25                       4,0
    (6)    Magnesiumstearat                   1,5
                                           268,0
```

Herstellung:

(1) wird mit (2) verrieben. Der Verreibung wird (3) und (4) zugemischt. Die Mischung wird mit einer alkoholischen Lösung von (5) granuliert. Dem getrockneten Granulat wird (6) zugemischt. Die fertige Mischung wird in Kapseln Größe 1 (268 mg/Kapsel) abgefüllt.

Bemerkung:

Wirkstoff und Säure liegen in ein und demselben Granulat zusammen vor.
Bestimmung der Auflösegeschwindigkeit: analog zu Beispiel 1a.

Ergebnis:

```
    Nach   5 min:   84,1 %
          10 min:   90,2 %
          15 min:   91,7 %
          30 min:   92,5 %   Pimobendan gelöst.
```

Beispiel 4

Kapseln zu 2,5 mg Pimobendan

Kapseln mit 209 mg Zitronensäure

Zusammensetzung:

1 Kapsel enthält (mg)

|     |                     |       |
|-----|---------------------|-------|
| (1) | Pimobendan          | 2,5   |
| (2) | Zitronensäure, pulv.| 209,0 |
| (3) | mikrokrist. Cellulose | 40,0 |
| (4) | Siliciumdioxid      | 11,0  |
| (5) | Polyvinylpyrrolidon | 4,0   |
| (6) | Magnesiumstearat    | 1,5   |
|     |                     | 268,0 |

Herstellung:

Analog zu Beispiel 3c.

Bemerkung:

Wirkstoff und Säure liegen in ein und demselben Granulat vor.
Bestimmung der Auflösegeschwindigkeit: analog zu Beispiel 1a.

Ergebnis:

Nach 15 min: 96,5 %
30 min: 99,1 % Pimobendan gelöst.

Beispeil 5

Filmtablette zu 2,5 mg Pimobendan

Tabletten mit 50 mg Zitronensäure

Zusammensetzung:

1 Tablette enthält (mg)

|     |                                      |       |
|-----|--------------------------------------|-------|
| (1) | Pimobendan                           | 2,5   |
| (2) | Zitronensäure pulv., wasserfrei      | 50,0  |
| (3) | Avicel® PH 101                       | 13,0  |
| (4) | Calciumhydrogenphosphat, wasserfrei  | 15,0  |
| (5) | Maisstärke, ungetr.                  | 6,0   |
| (6) | Kollidon® 25                         | 0,5   |
| (7) | Polyvinylpyrrolidon, unlöslich       | 59,0  |
| (8) | Compritol® 888                       | 3,0   |
| (9) | Magnesiumstearat                     | 1,0   |
|     |                                      | 150,0 |

Herstellung:

Die Herstellung erfolgte wie im Beispiel 2b beschrieben, die preßfertige Mischung wurde jedoch zu bikonvexen Tabletten verpreßt. Diese werden mit 5 mg Hydroxypropylmethylcellulose/Tablette überzogen.

Bemerkung:

Wirkstoff und Säure liegen gemeinsam im selben Granulat vor.
Bestimmung der Auflösegeschwindigkeit: analog zu Beispiel 1a.

Ergebnis:

```
Nach 10 min:  76,8 %
      30 min:  86,1 %  Pimobendan gelöst.
```

**Patentansprüche**

1. Oral zu applizierende Arzneimittelformen des Pimobendans enthaltend Pimobendan und Zitronensäure in inniger Vermischung in einem Gewichtsverhältnis von höchstens oder kleiner als 1:5 neben üblichen Hilfsstoffen.

2. Arzneimittelformen gemäß Anspruch 1 in Form von Granulaten, Pulvern oder Pellets, gegebenenfalls in Kapseln abgefüllt.

3. Arzneimittelformen gemäß Anspruch 2, wobei die Granulate, Pulver oder Pellets mit entsprechenden Hilfsstoffen zu Tabletten verpreßt sind, die gegebenenfalls mit einem geschmacksabdeckenden Überzug versehen sind.

4. Arzneimittelformen gemäß den Ansprüchen 1 bis 3, gekennzeichnet durch ein Gewichtsverhältnis von Pimobendan zu Zitronensäure zwischen 1 zu 10 und 1 zu 20.

5. Arzneimittelformen gemäß den Ansprüchen 1 bis 4 enthaltend als Hilfsstoffe ein Sprengmittel.

6. Verfahren zur Herstellung oral zu applizierender Arzneimittelformen des Pimobendans, dadurch gekennzeichnet, daß Pimobendan und Zitronensäure in einem Gewichtsverhältnis von höchstens oder kleiner als 1:5 innig vermischt und entweder nicht-wäßrig feucht granuliert werden oder wäßrig mit Hilfe entsprechender Granulierverfahren, die eine genau dosierte Zugabe der Granulierflüssigkeit unter gleichzeitiger Trocknung ermöglichen, oder mit Hilfe einer Trockengranulation.

7. Verfahren gemäß Anspruch 6, dadurch gekennzeichnet, daß die jeweiligen Granulate zu Pellets verarbeitet werden.

8. Verfahren gemäß Anspruch 6 und 7, dadurch gekennzeichnet, daß die Granulate oder Pellets mit entsprechenden Hilfsstoffen zu Tabletten verpreßt werden, die gegebenenfalls einen geschmacksabdeckenden Überzug erhalten.

9. Verfahren gemäß den Ansprüchen 6 und 7, dadurch gekennzeichnet, daß die Granulate und Pellets in Kapseln abgefüllt werden.

**Claims**

1. Pharmaceutical forms of pimobendan for oral administration, containing pimobendan and citric acid in an intimate mixture in a ratio by weight of at least or less than 1:5, together with conventional excipients.

2. Pharmaceutical forms according to claim 1 in the form of granulates, powders or pellets, optionally packed into capsules.

3. Pharmaceutical forms according to claim 2, the granulates, powders or pellets being compressed with suitable excipients into tablets which are optionally provided with a flavour-masking coating.

4. Pharmaceutical forms according to claims 1 to 3, characterised by a weight ratio of pimobendan to citric acid of between 1:10 and 1:20.

5. Pharmaceutical forms according to claims 1 to 4 containing, as excipient, a disintegrant.

6. Process for preparing pharmaceutical forms of pimobendan for oral administration, characterised in that pimobendan and citric acid are intimately mixed in a ratio by weight of at least or less than 1:5 and are either granulated non-aqueously moistened or are granulated with water by suitable granulating methods which allow accurately metered addition of the granulating liquid with simultaneous drying, or by means of dry granulation.

7. Process according to claim 6, characterised in that the granulates are processed into pellets.

8. Process according to claims 6 and 7, characterised in that the granulates or pellets are compressed with suitable excipients into tablets which are optionally given a flavour-masking coating.

9. Process according to claims 6 and 7, characterised in that the granulates and pellets are packed into capsules.

**Revendications**

1. Formes médicamenteuses du Pimobendane destinées à une application orale, contenant du Pimobendane et de l'acide citrique sous forme d'un mélange intime dans des proportions pondérales inférieures ou au plus égales à 1:5 ainsi que des adjuvants habituels.

2. Formes médicamenteuses selon la revendication 1 sous forme de granulés, poudres ou pastilles, éventuellement introduites dans des capsules.

3. Formes médicamenteuses selon la revendication 2, dans lesquelles les granulés, poudres ou pastilles sont compactées avec des adjuvants correspondants en comprimés qui sont munis éventuellement d'un revêtement masquant le goût.

4. Formes médicamenteuses selon les revendications 1 à 3, caractérisées par des proportions pondérales du Pimobendane à l'acide citrique comprises entre 1 et 10 et 1 et 20.

5. Formes médicamenteuses selon les revendications 1 à 4 contenant un agent d'expansion en tant qu'adjuvant.

6. Procédé de préparation de formes médicamenteuses du Pimobendane destinées à une application orale, caractérisé en ce que le Pimobendane et l'acide citrique sont mélangés intimement dans des proportions pondérales inférieures ou au plus égales à 1:5 et sont granulés à l'état humide sans eau ou en présence d'eau à l'aide de procédés de granulation correspondants qui permettent une addition exactement dosée du liquide de granulation avec un séchage simultané, ou à l'aide d'une granulation à sec.

7. Procédé selon la revendication 6, caractérisé en ce que les différents granulés sont transformés en pastilles.

8. Procédé selon les revendications 6 et 7, caractérisé en ce que les granulés ou les pastilles sont compactés avec des adjuvants correspondants en comprimés qui reçoivent éventuellement un revêtement masquant le goût.

9. Procédé selon les revendications 6 et 7, caractérisé en ce que les granulés et les pastilles sont introduits dans des capsules.

# FIG.1

Plasmaspiegel von Pimobendan 5 mg p.o., Tablette ohne
Zitronensäure, Hund, n = 5, MW ± S.E. (Beispiel 1a).

# FIG.2

Plasmaspiegel von Pimobendan 5 mg p.o., Tablette mit 50 mg
Zitronensäure, Hund, n = 5, MW ± S.E. (Beispiel 1b)

# FIG.3

UD-CG 115 BS Plasmaspiegel nach Gabe von 5 mg Pimobendan (Probanden).

# FIG.4

Mittlerer Plasmaspiegelverlauf von Pimobendan an jeweils den gleichen 4 Probanden nach oraler Gabe einer 2·5 mg Tablette (■) und einer Kapsel (●) (MW± S.E.)

# FIG.5

NG/ML    Proband a

(Beispiel 3a)
Pimobendan Plasmaspiegel an 3
Probanden nach oraler Gabe
von 5 mg
(ohne Säurezusatz)

NG/ML    Proband b

NG/ML    Proband c